# EUROPÄISCHE PATENTSCHRIFT

(11) **EP 4 387 693 B1**
(45) Veröffentlichungstag und Bekanntmachung des Hinweises auf die Patenterteilung: **25.06.2025**
(21) Anmeldenummer: 22834875.1
(22) Anmeldetag: 07.12.2022
(51) Int. Cl.: A61M 5/315, A61D 7/00, A61M 5/20

(54) **VETERINÄRMEDIZINISCHE SPRITZE**
VETERINARY SYRINGE
SERINGUE VÉTÉRINAIRE

(30) Priorität: 23.12.2021 DE 102021134597
(43) Veröffentlichungstag der Anmeldung: 26.06.2024
(73) Patentinhaber: Henke-Sass, Wolf GmbH, 78532 Tuttlingen (DE)
(72) Erfinder: EISELE, Melanie, 78573 Wurmlingen (DE)
(74) Vertreter: Patentanwälte Geyer, Fehners & Partner mbB
(86) Internationale Anmeldenummer: PCT/EP2022/084758
(87) Internationale Veröffentlichungsnummer: WO 2023/117424

(56) Entgegenhaltungen:
- NZ-A- 521 084
- US-A1- 2004 200 861
- US-A1- 2013 261 532
- US-B2- 6 554 161

## Beschreibung

Die vorliegende Erfindung betrifft eine veterinärmedizinische Spritze mit den Merkmalen des Anspruches 1, mit der z.B. Medikamente in unterschiedlich vorwählbaren Mengen verabreicht werden können.

Bei bekannten veterinärmedizinischen Spritzen besteht häufig die Schwierigkeit, dass die Bedienbarkeit zur Einstellung der unterschiedlichen vorwählbaren Mengen kompliziert ist, dass in unerwünschter Weise Verluste des Medikaments bei einer Umdosierung auftreten und dass die ergonomische Handhabbarkeit schlecht ist, weil z.B. eine Kolbenstange über den Handgriff nach hinten herausragt.

Aus der NZ 521 084 A ist eine veterinärmedizinische Spritze mit den Merkmalen des Oberbegriffs des Anspruches 1 bekannt. Die US 2004/200861 A1, US 2013/26153 A1 und die US 6 554 161 B2 zeigen jeweils eine veterinärmedizinische Spritze.

Aufgabe der vorliegenden Erfindung ist es daher, eine verbesserte veterinärmedizinische Spritze bereitzustellen.

Die Erfindung ist im Anspruch 1 definiert. Vorteilhafte Weiterbildungen sind in den abhängigen Ansprüchen angegeben.

Die veterinärmedizinische Spritze kann einen Grundkörper, der einen ein vorderes Abgabeende aufweisenden Spritzenzylinder zur Aufnahme von abzugebendem Fluid umfasst, eine Kolbenstange, die einen vorderen Endabschnitt mit einem Kolben, der im Spritzenzylinder entlang einer Verschieberichtung verschiebbar angeordnet ist, und einen hinteren Endabschnitt, der außerhalb des Spritzenzylinders positioniert ist, aufweist, einen Handgriff, der einen vorderen Griffteil am Grundkörper und einen hinteren Griffteil, der schwenkbar am vorderen Griffteil befestigt ist, aufweist, und eine Dosiereinrichtung zur Einstellung des abzugebenden Fluidvolumens aufweisen. Der hintere Endabschnitt der Kolbenstange kann schwenkbar am hinteren Griffteil (bevorzugt an einem zum vorderen Griffteil weisenden Abschnitt des hinteren Griffteils) befestigt sein, so dass durch Schwenken des hinteren Griffteils in Richtung zum Grundkörper hin die Kolbenstange und somit der Kolben im Spritzenzylinder zum vorderen Abgabeende bewegt wird, um im Spritzenzylinder vorhandenes Fluid abzugeben. Die Dosiereinrichtung kann am Grundkörper angeordnet sein und einen verstellbaren vorderen Anschlag für die Kolbenstange bereitstellen, der einen minimalen Abstand zwischen dem Kolben (z.B. der Kolbenfläche des Kolbens) und dem vorderen Abgabeende festlegt, der beim Bewegen des vorderen Endabschnitts der Kolbenstange im Spritzenzylinder zum vorderen Abgabeende erreicht werden kann. Dadurch ist festgelegt, wie weit in Richtung zum vorderen Abgabeende der Kolben maximal bewegbar ist. Der verstellbare vordere Anschlag der Dosiereinrichtung kann in mindestens zwei vorgegebene Einstellpositionen, in denen die Position des vorderen Anschlags in Verschieberichtung verschieden ist, positionierbar sein, so dass durch das Einstellen einer der Einstellpositionen der maximale Hub der Kolbenstange und damit das maximale abgebbare Fluidvolumen einstellbar ist.

Da der hintere Endabschnitt der Kolbenstange schwenkbar am hinteren Griffteil befestigt ist, steht die Kolbenstange nicht über den hinteren Griffteil vor, so dass der hintere Griffteil ergonomisch ausgebildet werden kann. Somit steht kein Teil der Kolbenstange (wie z.B. der hintere Endabschnitt der Kolbenstange) über die vom Grundkörper weg weisende Seite des hinteren Griffteils vor. Insbesondere kann die vom Grundkörper weg weisende Seite des hinteren Griffteils als durchgehende Fläche ausgebildet sein. Da der hintere Endabschnitt der Kolbenstange schwenkbar am hinteren Griffteil (bevorzugt an einem zum vorderen Griffteil weisenden Abschnitt des hinteren Griffteils) befestigbar ist, kann die vom Grundkörper weg weisende Seite des hinteren Griffteils insbesondere auch in dem Abschnitt, der der schwenkbaren Befestigung des hinteren Endabschnitts gegenüberliegt, als durchgehende Fläche ausgebildet sein. Ferner kann die vom Grundkörper weg weisende Seite des hinteren Griffteils ergonomisch geformt sein. Natürlich kann auch das vordere Griffteil ergonomisch ausgebildet sein.

Bevorzugt kann das hintere Griffteil relativ zum vorderen Griffteil um eine erste Schwenkachse verschwenkt werden. Die erste Schwenkachse kann sich senkrecht zur Verschieberichtung erstrecken. Der hintere Endabschnitt der Kolbenstange ist insbesondere so am hinteren Griffteil in einem Verbindungsbereich fixiert, dass nur ein relatives Verschwenken um eine zweite Schwenkachse möglich ist, die durch den Verbindungsbereich läuft. Bevorzugt ist dieser Verbindungsbereich an einem zum vorderen Griffteil weisenden Abschnitt des hinteren Griffteils ausgebildet. Die zweite Schwenkachse kann sich senkrecht zur Verschieberichtung und/oder parallel zur ersten Schwenkachse erstrecken.

Aufgrund der Verstellbarkeit des vorderen Anschlags in Verschieberichtung wird stets nur die Fluidmenge bzw. das Fluidvolumen ausgespritzt, das eingestellt wird. Beim nachfolgenden Auffüllen des Spritzzylinders liegt wieder ein vollständig gefüllter Spritzzylinder vor und ein Verstellen des vorderen Anschlags führt zu keinerlei Verlust des im Spritzenzylinder befindlichen Fluids. Bei dem Fluid kann es sich insbesondere um eine Flüssigkeit handeln. Das Fluid kann ein Medikament, ein Impfstoff oder ähnliches sein.

Die Dosiereinrichtung kann ein am Grundkörper drehbar gelagertes Drehelement aufweisen, das für jede der vorgegebenen Einspritzpositionen ein Anschlagelement (z.B. eine Rippe) umfasst. Die Anschlagelemente können in Drehrichtung des Drehelements voneinander beabstandet angeordnet sein, wobei durch Drehen des Drehelementes das gewünschte Anschlagelement als vorderer Anschlag positionierbar ist.

Damit kann in einfacher Art und Weise (lediglich durch Drehen des Drehelementes) das maximale abgebbare Fluidvolumen sicher eingestellt werden.

Die Anschlagelemente können auf einer wendelförmigen Grundbahn angeordnet sein, die einen Kanal umgibt, durch den sich die Kolbenstange erstreckt.

Damit ist eine kompakte Ausbildung der Dosiereinrichtung möglich.

Das Drehelement kann einen zylinderförmigen Rastabschnitt mit Nuten aufweisen, wobei jede Nut einem der Anschlagelemente zugeordnet ist und der Grundkörper ein Eingriffselement aufweist, das bei den vorgegebenen Einstellpositionen in Eingriff mit einer der Nuten steht. Damit kann sicher die gewünschte Anschlagposition gewählt und eingestellt werden.

Die Nuten des Rastabschnitts können auf einer äußeren Umfangsfläche des Drehelementes (z.B. eines Zylinderabschnittes) ausgebildet sein.

Die Kolbenstange kann einen Kolbenstangenanschlag aufweisen, der beim Erreichen des minimalen Abstands in Kontakt mit dem vorderen Anschlag steht. Der Kolbenstangenanschlag kann stegförmig ausgebildet sein und sich in Längsrichtung der Kolbenstange und radial erstrecken.

Der Kolbenstangenanschlag kann eine Anschlagfläche aufweisen, die beispielsweise konkav gekrümmt ist. In diesem Fall ist es bevorzugt, dass die von den Anschlagelementen bereitgestellten Anschlagflächen konvex gekrümmt sind, so dass dadurch ein sicherer Kontakt der beiden Anschlagflächen des Kolbenstangenanschlags einerseits und des entsprechend gewählten Anschlagelements andererseits gewährleistet ist. Natürlich ist es auch möglich, dass die Anschlagflächen der Anschlagelemente konkav ausgebildet sind und die Anschlagfläche des Kolbenstangenanschlags konvex ausgebildet ist. Ferner ist es möglich, dass die Anschlagflächen jeweils plan sind.

Der hintere Endabschnitt der Kolbenstange ist insbesondere so schwenkbar am hinteren Griffteil befestigt, dass eine relative Schwenkbewegung zwischen der Kolbenstange und dem hinteren Griffteil die einzig mögliche Relativbewegung zwischen der Kolbenstange und dem hinteren Griffteil ist. Damit ist z.B. eine Relativbewegung in und quer zur Längsrichtung der Kolbenstange zwischen der Kolbenstange und dem hinteren Griffteil nicht möglich. Eine solche schwenkbare Befestigung des hinteren Endabschnitts der Kolbenstange am hinteren Griffteil kann auch als fixe Kolbenstangenaufhängung bezeichnet werden.

Die Kolbenstange kann im Grundkörper (insbesondere der Kolben im Spritzenzylinder) so geführt sein, dass die Kolbenstange (bzw. der Kolben) nur entlang der Verschieberichtung verschiebbar bzw. bewegbar ist. Durch die fixe Kolbenstangenaufhängung kann bei einem Verschwenken des hinteren Griffteils hin zum vorderen Griffteil daher am hinteren Endabschnitt der Kolbenstange eine Bewegungskomponente quer zur Verschieberichtung (bzw. quer zur Längsrichtung der Kolbenstange) auftreten. Dies resultiert daraus, dass beim Verschwenken des hinteren Griffteils der Verbindungsbereich der schwenkbaren Befestigung am hinteren Griffteil auf einem Kreisbogen bewegt wird, wobei der Mittelpunkt des Kreisbogens auf der Schwenkachse der beiden Griffteile liegt. Die Kolbenstange kann daher insbesondere mit einer solchen Elastizität (oder z.B. Biegsamkeit) bereitgestellt werden, dass diese Bewegungskomponente quer zur Verschieberichtung kompensiert wird. Diese Kompensation kann somit insbesondere dadurch erreicht werden, dass die Bewegungskomponente quer zur Verschieberichtung zu einem Verbiegung oder Durchbiegen der Kolbenstange führt, was aufgrund der bereitgestellten Elastizität der Kolbenstange möglich ist.

Der Handgriff kann so ausgebildet sein, dass ein Federelement vorgesehen ist, das die beiden Griffteile auseinanderdrückt. Damit kann eine Grundstellung der veterinärmedizinischen Spritze bereitgestellt werden und beim Bedienen der Spritze muss ein Benutzer das hintere Griffteil gegen die Federkraft in Richtung zum vorderen Griffteil bewegen.

Insbesondere kann die Spritze (insbesondere der Grundkörper oder ein Hauptabschnitt des Grundkörpers) einen hinteren Anschlag aufweisen, der eine Bewegung der Kolbenstange entgegen der Verschieberichtung vom vorderen Abgabeende begrenzt. Die Position des hinteren Anschlags ist bevorzugt ortsfest und kann nicht verändert werden, so dass die Position des hinteren Anschlags die Grundstellung der Spritze (bzw. die maximal mögliche Verschwenkung des hinteren Griffteils zum vorderen Griffteil) festlegt.

Die erfindungsgemäße Spritze kann einen Anschluss für ein Fluidreservoir aufweisen, der in Fluidverbindung mit dem Spritzenzylinder steht. In der Fluidverbindung zum Spritzenzylinder kann ein Rückschlagventil angeordnet sein, das die Fluidverbindung sperrt und nur öffnet, wenn der Druck in der Fluidverbindung vor dem Rückschlagventil um einen vorbestimmten Wert über dem Druck im Spritzenzylinder liegt.

Der Anschluss zum Verbinden eines Fluidreservoirs kann am hinteren Griffteil ausgebildet sein. Vom Anschluss kann durch einen sich durch die Kolbenstange erstreckenden Zuführkanal eine Fluidverbindung bis zum Spritzenzylinder vorliegen.

Insbesondere kann dazu in der Kolbenstange (bevorzugt in ihrem vorderen Endabschnitt) ein Rückschlagventil vorgesehen sein, das erst öffnet, wenn der Druck im Zuführkanal um einen vorbestimmten Wert über dem Druck im Spritzenzylinder liegt.

Ferner kann das vordere Abgabeende über einen Abgabekanal mit einem vorderen Ende der Spritze in Fluidverbindung stehen und im Abgabekanal ein Rückschlagventil angeordnet sein, das erst öffnet, wenn der Druck im Zylinder einen vorbestimmten Wert (relativ zum Außendruck) übersteigt.

Damit ist die veterinärmedizinische Spritze bevorzugt als Selbstfüllerspritze ausgebildet, da nach Abgeben des vorbestimmten Fluidvolumens über das Rückschlagventil im Abgabekanal das hintere Griffteil durch die Federvorspannung der Feder wieder vom Abgabeende weg bewegt wird. Dies führt auch zu einer entsprechenden Bewegung des Kolbens, wodurch im Spritzenzylinder ein Unterdruck erzeugt wird, der zu einem Öffnen des Rückschlagventils in der Kolbenstange führt, so dass aufgrund der nun freien Fluidverbindung vom Spritzenzylinder bis zum Fluidreservoir Fluid aus dem Fluidreservoir in den Spritzenzylinder gesaugt werden kann. Sobald die Kolbenstange sich bis zum hinteren Anschlag der Spritze bewegt hat, schließt das Rückschlagventil in der Kolbenstange wieder und der Spritzenzylinder ist wieder vollständig gefüllt.

Es ist jedoch auch jede andere Art der Ausbildung als die Selbstfüllerspritze möglich. So kann auch ein Anschluss für ein Fluidreservoir am Grundkörper oder am Spritzenzylinder selbst vorgesehen sein, der über ein Rückschlagventil verschlossen ist, das sich nur bei Unterdruck im Spritzenzylinder öffnet. In diesem Fall muss in der Kolbenstange kein Zuführkanal ausgebildet sein.

Bei der veterinärmedizinischen Spritze kann der Kolben lösbar mit dem vorderen Endabschnitt verbunden sein, wobei nach Lösen des Kolbens vom Endabschnitt das Rückschlagventil zu Wartungszwecken (wie z.B. zum Reinigen) zugänglich ist. Ferner kann eine betätigbare Sicherung ausgebildet sein, die ein unbeabsichtigtes Lösen des Kolbens dadurch verhindert, dass ein Lösen des Kolbens vom vorderen Endabschnitt erst nach Betätigen der Sicherung möglich ist.

Die lösbare Verbindung kann z.B. eine Schraubverbindung, eine Bajonettverbindung oder eine sonstige Verbindung sein.

Die Sicherung kann als Verdrehsicherung ausgebildet sein, die ein Drehen (z.B. Abschrauben) des Kobens relativ zur Kolbenstange (bevorzugt um die Längsachse der Kolbenstange) blockiert.

Die Sicherung kann eine Sicherungsausnehmung als erstes Sicherungselement und eine Sicherungsnase als zweites Sicherungselement aufweisen, wobei zur Sicherung die Sicherungsnase in die Sicherungsausnehmung einsteht (die Sicherungsnase steht in Eingriff mit der Sicherungsausnehmung) und dadurch ein Lösen des Kolbens vom vorderen Endabschnitt verhindert. Eines der beiden Sicherungselemente kann am Kolben und das andere der beiden Sicherungselemente kann am vorderen Endabschnitt ausgebildet sein.

Die Sicherungsausnehmung kann z.B. als Durchgangsöffnung in einer entsprechenden Wandung oder in Art eines Sackloches (als sich nicht durch die gesamte Dicke der Wandung erstreckend) ausgebildet sein.

Der Kolben kann einen Kolbenabschnitt mit einer Kolbenfläche und einen hohlzylinderförmigen Befestigungsabschnitt aufweisen, wobei das erste Sicherungselement an der Wandung des hohlzylinderförmigen Befestigungsabschnitts ausgebildet ist. Das erste Sicherungselement kann z.B. am vom Kolbenabschnitt wegweisenden Ende des Befestigungsabschnitts ausgebildet sein. Ferner kann der hohlzylinderförmige Befestigungsabschnitt ein Innengewinde aufweisen und kann der vordere Endabschnitt ein mit dem Innengewinde zusammenwirkendes Außengewinde aufweisen.

Das Innengewinde kann mindestens einen gewindefreien Bereich aufweisen, der sich in Längsrichtung des hohlzylinderförmigen Befestigungsabschnitts (bevorzugt geradlinig) erstreckt. Das Innengewinde kann somit auch als unterbrochenes Innengewinde bezeichnet werden. Bevorzugt erstreckt sich der mindestens eine gewindefreie Bereich im Endbereich des Innengewindes am vom Kolbenabschnitt wegweisenden Ende des Innengewindes. Der gewindefreie Bereich kann sich auch über die gesamte Länge des Innengewindes erstrecken.

Das Innengewinde kann zwei der gewindefreien Bereiche aufweisen, die dann bevorzugt einander gegenüberliegen.

Der mindestens eine gewindefreie Bereich des Innengewindes ist bevorzugt in Umfangsrichtung relativ zum ersten Sicherungselement versetzt angeordnet.

Der hohlzylinderförmige Befestigungsabschnitt ist bevorzugt so ausgebildet, dass er elastisch und deformierbar ist.

Das Außengewinde kann mindestens einen gewindefreien Bereich aufweisen, der sich in Längsrichtung der Kolbenstange (bevorzugt geradlinig) erstreckt. Das Außengewinde kann somit auch als unterbrochenes Außengewinde bezeichnet werden. Bevorzugt erstreckt sich der mindestens eine gewindefreie Bereich im Endbereich des Außengewindes am zum Kolbenabschnitt weisenden Ende des Außengewindes. Der gewindefreie Bereich kann sich auch über die gesamte Länge des Außengewindes erstrecken.

Das Außengewinde kann zwei der gewindefreien Bereiche aufweisen, die dann bevorzugt einander gegenüberliegen.

Insbesondere kann der mindestens eine gewindefreie Bereich des Außengewindes in Umfangsrichtung relativ zum zweiten Sicherungselement versetzt angeordnet sein.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine Schnittansicht einer Ausführungsform der erfindungsgemäßen veterinärmedizinischen Spritze 1;
- Fig. 2: eine isometrische Ansicht der Kolbenstange 8 samt Kolben K;
- Fig. 3: eine Vorderansicht von Kolbenstange 8 und Kolben K;
- Fig. 4: eine Schnittansicht der Kolbenstange 8 samt Kolben K entlang der Schnittlinie A-A in Fig. 3;
- Fig. 5: eine vergrößerte Ansicht des Details C von Fig. 4;
- Fig. 6: eine Schnittansicht der Kolbenstange 8 samt Kolben K entlang der Schnittlinie B-B in Fig. 4;
- Fig. 7: eine isometrische Ansicht der Kolbenstange 8;
- Fig. 8: eine Vorderansicht der Kolbenstange 8 von Fig. 7;
- Fig. 9: eine Schnittansicht der Kolbenstange 8 entlang der Schnittlinie A-A in Fig. 8;
- Fig. 10: eine isometrische Ansicht des Kolbens K;
- Fig. 11: eine Vorderansicht des Kolbens K;
- Fig. 12: eine Schnittansicht des Kolbens K entlang der Schnittlinie A-A in Fig. 11;
- Fig. 13: eine Draufsicht des Verbindungsstücks 23;
- Fig. 14: eine isometrische Ansicht des Verbindungsstücks 23;
- Fig. 15: eine Vorderansicht des Verbindungsstücks 23;
- Fig. 16: eine Schnittansicht des Verbindungsstücks 23 entlang der Schnittlinie A-A in Fig. 15;
- Fig. 17: eine Vorderansicht des hinteren Griffteils 15;
- Fig. 18: eine Schnittansicht des hinteren Griffteils 15 entlang der Schnittlinie A-A in Fig. 17;
- Fig. 19: eine isometrische Ansicht des hinteren Griffteils 15;
- Fig. 20: eine isometrische Ansicht des Drehelements 51;
- Fig. 21: eine Draufsicht des Drehelements 51, und
- Fig. 22: eine Schnittansicht des Drehelements 51 von Fig. 20 und 21.

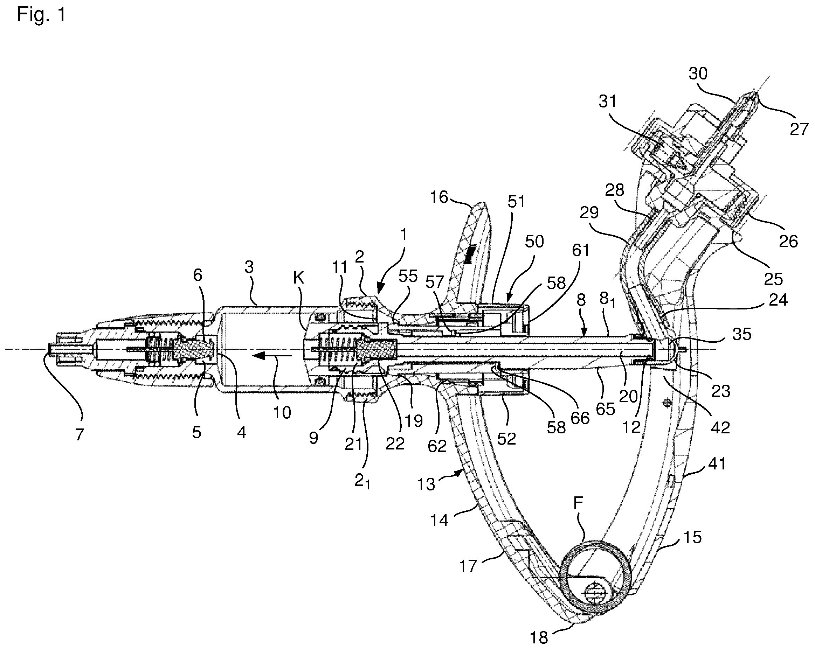

Bei der in Fig. 1 gezeigten Ausführungsform umfasst die erfindungsgemäße veterinärmedizinische Spritze 1 einen Grundkörper 2 mit einem Spritzenzylinder 3 zur Aufnahme von abzugebendem Fluid (z.B. ein Medikament oder Impfstoff in flüssiger Form). Der Spritzenzylinder 3 ist hier mittels einer Schraubverbindung lösbar mit einem Hauptabschnitt 2₁ des Grundkörpers 2 verbunden und umfasst ein vorderes Abgabeende 4, das in einen Abgabekanal 5 mündet, in dem ein erstes Rückschlagventil 6 sitzt. Am vorderen Ende 7 des Abgabekanals 5 kann z.B. eine nicht gezeigte Kanüle aufgesteckt werden.

Die Spritze 1 umfasst ferner eine Kolbenstange 8, die mit ihrem vorderen Endabschnitt 9 im Spritzenzylinder 3 entlang einer Verschieberichtung 10 verschiebbar angeordnet ist. Am vorderen Endabschnitt 9 ist ein Kolben K angeordnet, der beim Verschieben in Richtung zum vorderen Abgabeende 4 hin eine Abgabe von Fluid aus dem Spritzenzylinder 3 über das vordere Abgabeende 4 und den Abgabekanal 5 bewirkt, da aufgrund des durch die Kolbenbewegung im Spritzenzylinder 3 aufgebauten Druckes das erste Rückschlagventil 6 öffnet.

Die Kolbenstange 8 erstreckt sich über ein hinteres Ende 11 des Spritzenzylinders 3 aus dem Grundkörper 2 heraus und ist, wie nachfolgend noch im Detail beschrieben wird, mit ihrem hinteren Endabschnitt 12 gelenkig mit einem hinteren Griffteil 15 eines Handgriffs 13 der Spritze 1 verbunden.

Der Handgriff 13 weist ein am Grundkörper 2 (hier im Hauptabschnitt 2₁) ausgebildetes vorderes Griffteil 14 und das hintere Griffteil 15 auf. Das vordere Griffteil 14 weist einen nach oben vom Grundkörper 2 vorstehenden oberen Abschnitt 16 sowie einen nach unten vom Grundkörper 2 vorstehenden unteren Abschnitt 17 auf. Am freien Ende 18 des unteren Abschnitts 17 des vorderen Griffteils 14 ist das hintere Griffteil 15 um eine erste Schwenkachse (die hier senkrecht zur Zeichenebene von Fig. 1 verläuft) verschwenkbar montiert, wobei eine zwischen den beiden Griffteilen 14 und 15 vorgesehene Feder F die beiden Griffteile 14 und 15 auseinanderdrückt. Wie in der Schnittdarstellung von Fig. 1 gut erkennbar ist, weist der Handgriff 13 im Wesentlichen eine V-Form auf.

In der in Fig. 1 gezeigten Grundstellung der Spritze 1 liegt der vordere Endabschnitt 9 der Kolbenstange 8 an einem hinteren Anschlag 19, der im Hauptabschnitt 2₁ ausgebildet ist, an und kann daher nicht weiter weg vom vorderen Abgabeende 4 entgegen der Verschieberichtung 10 bewegt werden. Aufgrund der Feder F liegt diese Grundstellung vor, wenn keine externe Kraft auf den hinteren Griffteil 15 ausgeübt wird.

Wie insbesondere den Fig. 1, 4, 5, 6 und 9 entnommen werden kann, umfasst die Kolbenstange 8 einen Kolbenstangenhauptabschnitt 8₁ mit einem in Längsrichtung der Kolbenstange 8 durchgehenden Zuführkanal 20, der über einen Verbindungskanal 21 im vorderen Endabschnitt 9 sowie einer Durchgangsöffnung 70 im Kolben K in Fluidverbindung mit dem Inneren des Spritzenzylinders 3 steht, wobei im Verbindungskanal 21 ein zweites Rückschlagventil 22 angeordnet ist.

Um das zweite Rückschlagventil 22 im vorderen Endabschnitt 9 einbauen zu können und zu Wartungszwecken beispielsweise säubern zu können, ist der Kolben K über eine Schraubverbindung lösbar am vorderen Endabschnitt 9 befestigt.

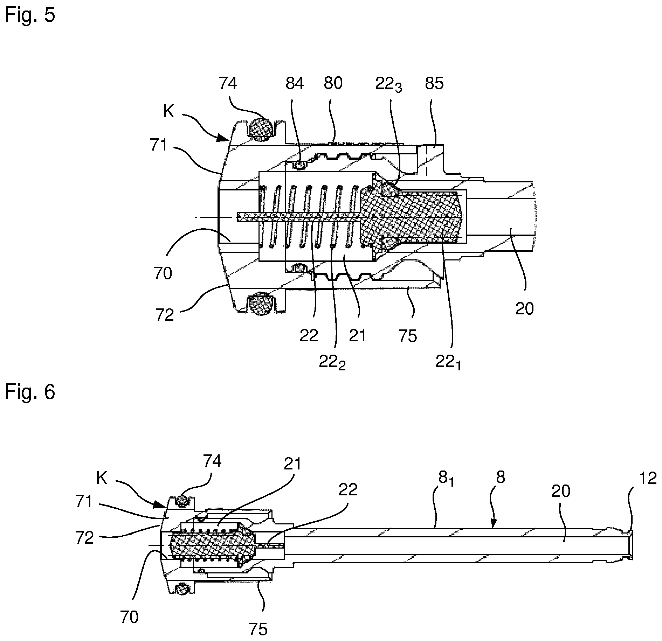

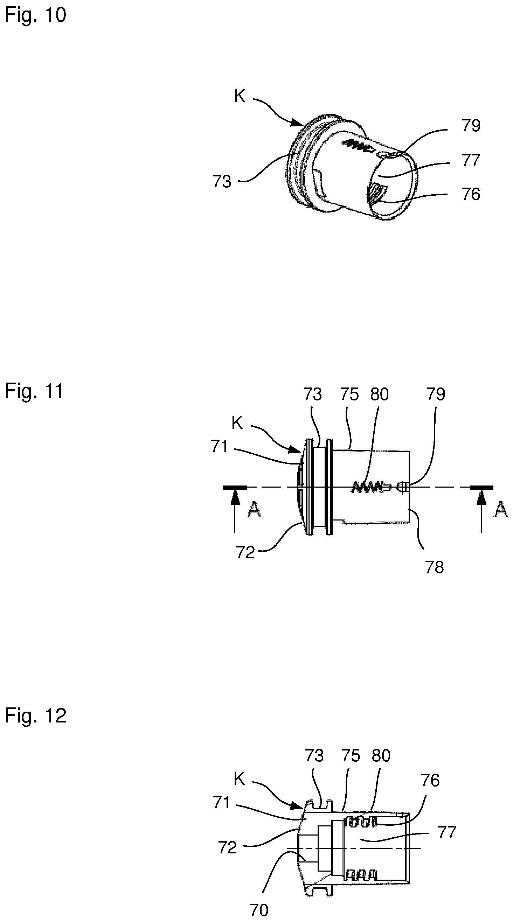

Im Detail umfasst der Kolben K, wie insbesondere Fig. 5 zu entnehmen ist, einen Kolbenabschnitt 71 mit einer Kolbenfläche 72 und einer ringförmigen Kolbennut 73, in der ein Kolbendichtring 74 sitzt. An den Kolbenabschnitt 71 schließt ein hohlzylinderförmiger Befestigungsabschnitt 75 an, der ein Innengewinde 76 aufweist, das in Umfangsrichtung nicht durchgehend ausgebildet ist, sondern zwei gegenüberliegende gewindefreie Bereiche 77 aufweist (Fig. 10 und 12).

In der Wandung des hohlzylinderförmigen Befestigungsabschnittes 75 ist am vom Kolbenabschnitt 71 weg weisenden Ende 78 eine Sicherungsausnehmung 79 (bzw. erstes Sicherungselement) ausgebildet. Ferner ist auf der Wandung des hohlzylinderförmigen Befestigungsabschnitts 75 noch eine Markierung 80 vorgesehen, die anzeigt, wie das zweite Rückschlagventil 22 in den Verbindungskanal 21 einzusetzen ist.

Da sich die Durchgangsöffnung 70 durch den gesamten Kolbenabschnitt 71 bis zum Inneren des hohlzylinderförmigen Befestigungsabschnitt 75 erstreckt, kann die gewünschte Fluidverbindung zum Zuführkanal 20 zum Inneren des Spritzzylinders 3 bereitgestellt werden, wenn der Kolben K auf den vorderen Endabschnitt 9 aufgeschraubt ist.

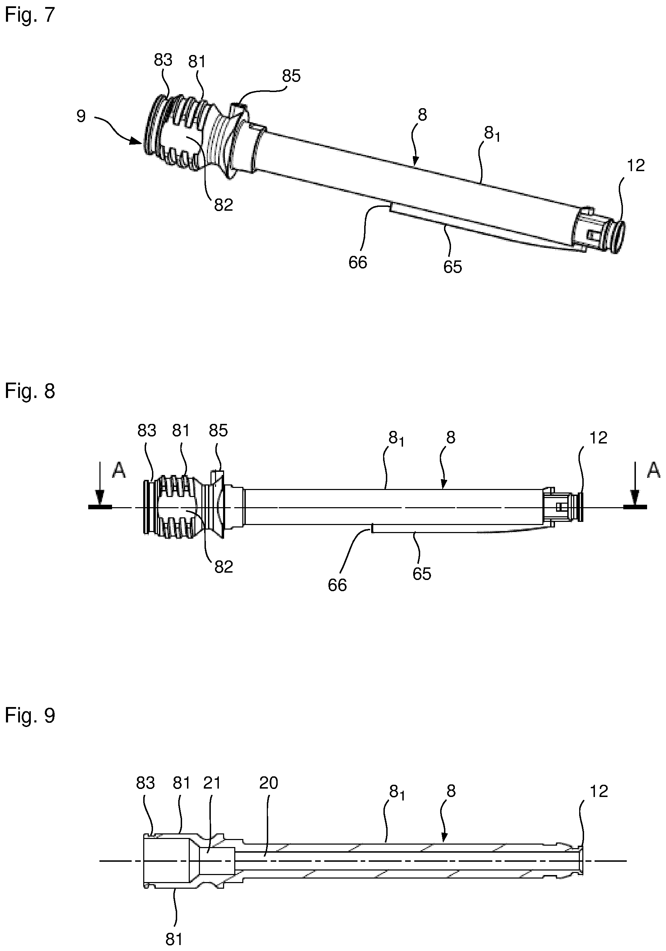

Zum Aufschrauben ist auf der Außenseite des vorderen Endabschnitts 9 ein Außengewinde 81 ausgebildet, das in Umfangsrichtung durch zwei gewindefreie Bereiche 82, die bevorzugt gegenüberliegen, unterbrochen ist (Fig. 7-9). Am vorderen Ende des vorderen Endabschnitts 9 ist eine Ringnut 83 ausgebildet, in der ein Dichtring 84 sitzt (Fig. 5).

Ferner umfasst der vordere Endabschnitt 9 eine radial vorstehende Sicherungsnase 85 (bzw. zweites Sicherungselement), die in Richtung zum hinteren Endabschnitt 12 der Kolbenstange 8 vom Außengewinde 81 beabstandet ist (Fig. 7 und 8).

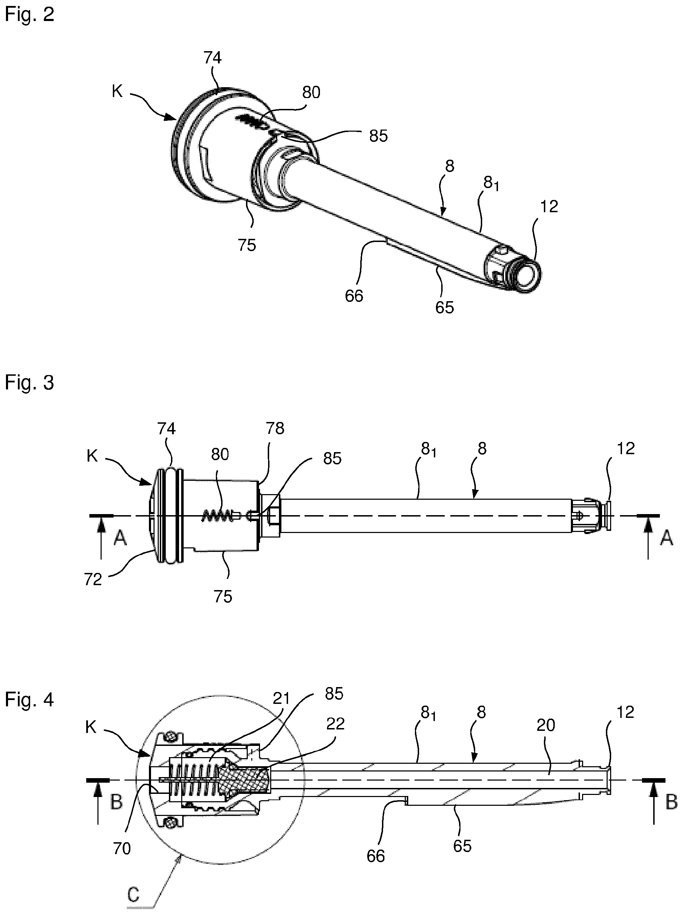

Die Abmessung der gewindefreien Bereiche 82 in Umfangsrichtung des Außengewindes 81 ist in Verbindung mit der Abmessung der gewindefreien Bereiche 77 des Innengewindes 76 des Kolbens K so gewählt, dass ein Aufschrauben des Kolben K auf den vorderen Endabschnitt 9 und eine dauerhafte Gewindeverbindung möglich sind. Dazu muss lediglich, bevor beim Aufschrauben das Ende 78 des hohlzylinderförmigen Befestigungsabschnitts 75 gegen die Sicherungsnase 85 stoßen würde, der hohlzylinderförmige Befestigungsabschnitt 75 in den Bereichen der gewindefreien Bereiche 77 zusammengedrückt werden. Aufgrund der dadurch bedingten Verformung des Befestigungsabschnitts 75 kann das Ende 78 des hohlzylinderförmigen Befestigungsabschnitts 75 über die Sicherungsnase 85 beim Aufschrauben bewegt und die Sicherungsausnehmung 79 so positioniert werden, dass nach Beenden des beschriebenen Zusammendrückens sich der hohlzylinderförmige Befestigungsabschnitt 75 aufgrund seiner Elastizität in seine Grundform entspannt (die Verformung des hohlzylinderförmigen Befestigungsabschnitts 75 wird somit rückgängig gemacht). Da der Kolben K so positioniert ist, dass die Sicherungsausnehmung 79 gegenüber der Sicherungsnase 85 liegt, steht dann die Sicherungsnase 85 in die Sicherungsausnehmung 79 ein, wie in Fig. 2 bis 6 gezeigt ist. Somit greift die Sicherungsnase 85 in die Sicherungsausnehmung 79 ein, wodurch ein Verdrehschutz bzw. eine Fixierung gegen Verdrehung vorliegt.

Selbst wenn auf den Kolben K eine Drehkraft ausgeübt wird, kann ein unerwünschtes Abschrauben des Kolbens K vom vorderen Endabschnitt 9 sicher verhindert werden, da die Sicherungsnase 85 in die Sicherungsausnehmung 79 einsteht und somit ein Abschrauben des Kolbens K vom vorderen Endabschnitt 9 sicher verhindert ist. Eine unerwünschte Drehkraft auf den Kolben K kann beispielsweise beim Abschrauben des Zylinders 3 vom Hauptabschnitt 2₁ des Grundkörpers 2 zu Reinigungszwecken auftreten, wenn z.B. aufgrund einer Verschmutzung die Reibung zwischen Zylinder 3 und Kolben K höher ist als die Reibung zwischen Kolben K und vorderem Endabschnitt 9.

Wenn nach einer Benutzung der erfindungsgemäßen Spritze 1 das zweite Rückschlagventil 22 gereinigt oder gewartet werden muss, kann zunächst der Zylinder 3 vom Hauptabschnitt 2₁ des Grundkörpers 2 abgeschraubt werden. Aufgrund der Verdrehsicherung mittels der Sicherungsnase 85 und der Sicherungsausnehmung 79 bleibt dabei der Kolben K auf dem vorderen Endabschnitt 9. Um nun den Kolben K vom vorderen Endabschnitt 9 zu lösen, muss der hohlzylinderförmige Befestigungsabschnitt 75 durch Zusammendrücken im Bereich der zwei gegenüberliegenden gewindefreien Bereiche 77 deformiert werden, so dass die Querschnittsform des hohlzylinderförmigen Befestigungsabschnitts 75 im Bereich der Sicherungsausnehmung 79 nicht mehr kreisförmig sondern oval ist. Dadurch wird die Sicherungsausnehmung 79 entlang der radialen Erstreckungsrichtung der Führungsnase 85 vom vorderen Endabschnitt 9 weg bewegt, so dass kein Eingriff der Sicherungsnase 85 mehr mit der Sicherungsausnehmung 79 vorliegt. In diesem Zustand kann mit dem Abschrauben des Kolbens K begonnen werden. Die Abmessung der Sicherungsnase 85 sowie der Sicherungsausnehmung 79 in Längsrichtung der Kolbenstange 8 sind in der beschriebenen Ausführungsform so gewählt, dass nach einer vollständigen Drehung (360°) des Kolbens K das Ende 78 der Wandung des hohlzylinderförmigen Befestigungsabschnittes 75 die Sicherungsnase 85 gerade nicht mehr berührt. Somit kann der Kolben K weiter abgeschraubt werden, da keine Verdrehsicherung mehr vorliegt.

Nach Abschrauben des Kolbens K ist das zweite Rückschlagventil 22 mit seinem Ventilkörper 22₁, seiner Ventilfeder 22₂ und seiner Dichtung 22₃ frei zugänglich und kann gereinigt und gewartet werden.

Danach kann der Kolben K in der bereits beschriebenen Art und Weise wieder aufgeschraubt werden, so dass die gewünschte Verdrehsicherung durch Eingriff der Sicherungsnase 85 in der Sicherungsausnehmung 79 vorliegt.

Der hintere Endabschnitt 12 der Kolbenstange 8 ist mit einem im hinteren Griffteil 15 drehbar gelagerten Verbindungsstück 23 (Fig. 1 und Fig. 13-16) verbunden, das einen Schlauchansatz 24 aufweist.

Am oberen freien Ende 25 (Fig. 1, 17 bis 19) des hinteren Griffteils 15 ist ein Flaschenaufsatz 26 mit einem Einstichdorn 27 (Fig. 1) montiert, der beispielsweise durch eine Membran einer am Flaschenaufsatz 26 befestigten Medikamentenflasche (nicht gezeigt) stechen kann, wenn die Medikamentenflasche am Flaschenaufsatz 26 befestigt wird. Der Flaschenaufsatz 26 weist ferner einen Schlauchansatz 28 auf, der in Fluidverbindung mit dem Einstichdorn 27 steht. Auf die beiden Schlauchansätze 28 und 24 sind zwei Enden eines Schlauches 29 aufgeschoben, so dass über das Verbindungsstück 23 eine Fluidverbindung vom Einstichdorn 27 (und somit zum Inhalt der entsprechenden Medikamentenflasche) zum Zuführkanal 20 der Kolbenstange 8 besteht.

Der Flaschenaufsatz 26 weist noch einen im Einstichdorn 27 verlaufenden Belüftungskanal 30, der mit der Umgebung über ein drittes Rückschlagventil 31 verbunden ist, auf, um die Ausbildung eines zu großen Unterdrucks in der Medikamentenflasche bei der Entnahme der Flüssigkeit zu vermeiden.

Die Spritze 1 ist als sogenannte Selbstfüllerspritze ausgebildet, die nach einem erfolgten Ausspritzvorgang den Spritzenzylinder 3 automatisch mit der Menge des ausgespritzten Fluids auffüllt.

So kann ein Benutzer der Spritze 1 den Handgriff 13 so in seiner Hand halten, dass sein Zeigefinger am oberen Abschnitt 16, sein Mittelfinger, sein Ringfinger und sein kleiner Finger am unteren Abschnitt 17 des vorderen Griffteils 14 sowie der Handballen mit Daumen am hinteren Griffteil 15 anliegen. Wenn der Benutzer nun seine Hand zusammendrückt, bewegt er den hinteren Griffteil 15 gegen die Kraft der Feder F in Richtung zum Grundkörper 2, wodurch die Kolbenstange 8 mit ihrem vorderen Endabschnitt 9 und somit mit dem Kolben K im Spritzenzylinder 3 entlang der Verschieberichtung 10 zum vorderen Abgabeende 4 bewegt wird. Dadurch baut sich ein Überdruck im Spritzenzylinder 3 auf, der dazu führt, dass das erste Rückschlagventil 6 öffnet und somit Fluid über den Abgabekanal 5 abgegeben werden kann. Wenn der Benutzer seine Hand wieder öffnet, wird das hintere Griffteil 15 aufgrund der Federkraft der Feder F vom Grundkörper 2 weg bewegt, wodurch die Kolbenstange 8 samt vorderen Endabschnitt 9 und Kolben K entgegen der Verschieberichtung 10 im Spritzenzylinder 3 bewegt wird. Dadurch schließt das erste Rückschlagventil 6 und es baut sich ein Unterdruck im Inneren des Spritzenzylinders 3 auf, der dazu führt, dass sich das zweite Rückschlagventil 22 im vorderen Endabschnitt 9 öffnet und dadurch Fluid aus der Medikamentenflasche in das Innere des Spritzenzylinders 3 gesaugt wird. Die Rückwärtsbewegung des hinteren Griffteils 15 endet, wenn der vordere Endabschnitt 9 am hinteren Anschlag 19 anliegt. Das zweite Rückschlagventil 22 schließt wieder und das Innere des Spritzenzylinders 3 ist wieder vollständig mit abzugebendem Fluid gefüllt.

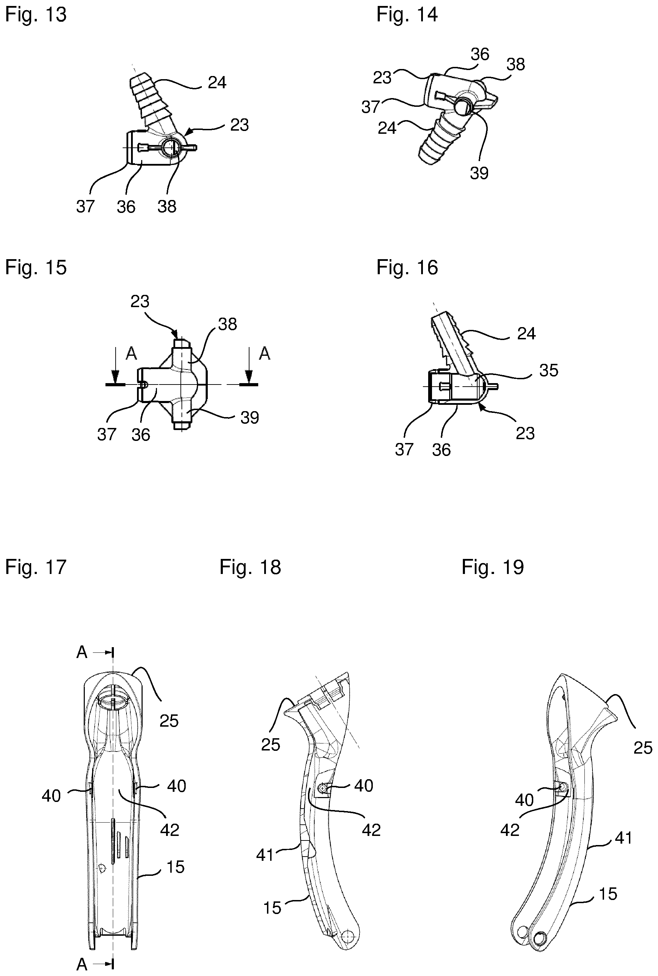

Wie in Fig. 13 bis 16 gezeigt ist, ist ein im Verbindungsstück 23 ausgebildete Kanal 35 im Wesentlichen V-förmig, wobei das Verbindungsstück 23 ein Grundelement 36 mit einem Anschluss 37 für den hinteren Endabschnitt 12 der Kolbenstange 8 sowie den sich dazu schräg erstreckenden Schlauchansatz 24 aufweist. Am Grundelement 36 sind zwei sich seitlich erstreckende Drehzapfen 38, 39 ausgebildet, die in entsprechenden Aufnahmen 40 (Fig. 17 - 19) des hinteren Griffteils 15 eingesetzt sind, so dass das Verbindungsstück 23 drehbar gelagert ist.

Durch diese Ausgestaltung der Spritze 1 ist somit der hintere Endabschnitt 12 der Kolbenstange 8 so drehbar am hinteren Griffteil 15 gelagert, dass eine longitudinale oder transversale Relativbewegung zwischen Kolbenstange 8 und hinterem Griffteil 15 nicht möglich ist. Man kann auch sagen, dass der hintere Endabschnitt 12 der Kolbenstange 8 so am hinteren Griffteil 15 in einem Verbindungsbereich fixiert ist, dass nur ein relatives Verschwenken um eine zweite Schwenkachse möglich ist, die durch den Verbindungsbereich läuft. Wie insbesondere Fig. 17 -19 in Verbindung mit Fig. 1 zu entnehmen ist, ist dieser Verbindungsbereich an einem zum vorderen Griffteil 14 weisenden Abschnitt 42 des hinteren Griffteils15 ausgebildet.

Bevorzugt erstreckt sich die zweite Schwenkachse senkrecht zur Verschieberichtung und/oder parallel zur ersten Schwenkachse, um die das hintere Griffteil 15 relativ zum vorderen Griffteil 14 verschwenkt werden kann. Durch diese Ausbildung der Befestigung des hinteren Endabschnitts 12 der Kolbenstange 8 am hinteren Griffteil 15 kann die vom Grundkörper 2 weg weisende hintere Seite 41 des hinteren Griffteils 15 ergonomisch zur besseren Handhabung der Spritze 1 ausgebildet sein kann. So kann die hintere Seite 41 und damit auch insbesondere der dem Verbindungsbereich gegenüberliegende Abschnitt der hinteren Seite 41 durchgehend (ohne Lücken oder Löcher) ausgebildet und eine entsprechende ergonomische Form für die Hand eines Benutzers aufweisen.

Da der hintere Endabschnitt 12 der Kolbenstange 8 so am hinteren Griffteil 15 im Verbindungsbereich fixiert ist, dass nur ein relatives Verschwenken um die zweite Schwenkachse möglich, wird die Kolbenstange 8 mit einer vorbestimmten Elastizität bereitgestellt, die ein Durchbiegen der Kolbenstange 8 zulässt. So kann bei einem Verschwenken des hinteren Griffteils 15 hin zum vorderen Griffteil 14 eine Bewegungskomponente quer zur Verschieberichtung (bzw. quer zur Längsrichtung der Kolbenstange) auftreten (in Fig. 1 z.B. von unten nach oben). Dies resultiert daraus, dass beim Verschwenken des hinteren Griffteils 15 der Verbindungsbereich der schwenkbaren Befestigung am hinteren Griffteil 15 auf einem Kreisbogen bewegt wird, wobei der Mittelpunkt des Kreisbogens auf der Schwenkachse der beiden Griffteile 14, 15 liegt. Aufgrund der vorbestimmten Elastizität der Kolbenstange 8 führt die Bewegungskomponente quer zur Verschieberichtung zu einem Verbiegen der Kolbenstange 8, so dass die Bewegungskomponente quer zur Verschieberichtung kompensiert wird. Die Elastizität ist dabei so ausgelegt, dass nur eine solche Durchbiegung der Kolbenstange 8 auftritt, die die Bewegung der Kolbenstange 8 mit ihrem vorderen Endabschnitt 9 und somit mit dem Kolben K im Spritzenzylinder 3 entlang der Verschieberichtung 10 zum vorderen Abgabeende 4 nicht beinflusst, so dass das Verschieben des Kolbens K im Spritzenzylinder 3 problemlos stattfindet.

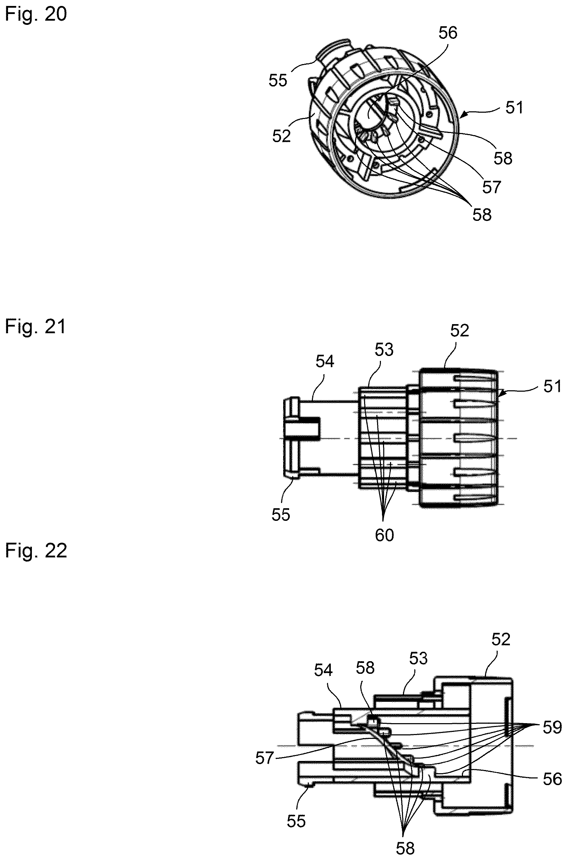

Die Spritze 1 weist ferner eine am Grundkörper 2 angeordnete Dosiereinrichtung 50 auf, mit der das maximale abgebbare Fluidvolumen verstellbar und einstellbar ist. Dazu umfasst die Dosiereinrichtung 50 ein am hinteren Ende des Grundkörpers 2 angeordnetes Drehelement 51 mit einer Endkappe 61 auf (Fig. 1). Das Drehelement 51 umfasst einen zylinderförmigen Griffabschnitt 52, einen daran anschließenden zylinderförmigen Rastabschnitt 53 und einem daran anschließenden zylinderförmigen Innenabschnitt 54 mit Rastnasen 55, wie insbesondere in Figuren 20 bis 22 gut erkennbar ist, wobei in den Figuren 20 bis 22 die Endkappe 61 nicht gezeigt ist. Im Inneren des Drehelements 51 ist ein zylinderförmiger Kanal 56 ausgebildet, durch den die Kolbenstange 8 läuft und geführt ist. Um den Kanal 56 herum sind auf einer wendelförmigen Grundbahn 57 in Umfangsrichtung voneinander beabstandete Anschlagelemente 58 (z.B. Rippen 58) ausgebildet, die hier bevorzugt die gleiche Höhe aufweisen, so dass ihre von der Grundbahn 57 wegweisende Anschlagflächen 59 unterschiedliche Abstände in Verschieberichtung 10 zum vorderen Abgabeende 4 aufweisen.

Auf der Außenseite des zylinderförmigen Rastabschnitts 53 ist für jedes Anschlagelement 58 eine in Längsrichtung verlaufende Nut 60 vorgesehen.

Im eingesetzten Zustand, der in Fig. 1 gezeigt ist, ist das Drehelement 51 drehbar am Grundkörper 2 gelagert, wobei sich die Kolbenstange 8 durch den Kanal 56 erstreckt.

Der Hauptabschnitt 2₁ des Grundkörpers 2 weist im Bereich des zylinderförmigen Rasterabschnitts 53 ein vorstehendes Eingriffselement 62 auf, das je nach Drehposition des Drehelements 51 mit einer der Längsnuten 60 in Eingriff steht.

Die Kolbenstange 8 weist, wie z.B. in Fig. 1, 2, 4, 7 und 8 ersichtlich ist, einen sich radial vom Kolbenstangenhauptabschnitt 8₁ erstreckenden Kolbenstangenanschlag 65 mit einer Anschlagfläche 66 auf. Die Kolbenstange 8 kann nun durch Bewegen des hinteren Griffteils 15 so lange in Richtung bis zum vorderen Abgabeende 4 bewegt werden, bis die Anschlagfläche 66 des Kolbenstangenanschlags 65 gegen die Anschlagfläche 59 des entsprechenden Anschlagelements 58 auf der wendelförmigen Grundbahn 57 in Kontakt kommt. Damit ist der maximale Hub der Kolbenstange 8 für einen Ausspritzvorgang festgelegt, wodurch auch das maximale Ausspritzvolumen des Fluids im Inneren des Spritzenzylinders 3 definiert ist.

Wenn die Spritze 1 in der in Fig. 1 gezeigten Grundstellung ist, kann durch Drehen des Drehelements 51 durch Betätigen des Griffabschnitts 52 das gewünschte maximale Ausspritzvolumen festgelegt werden. Dieses ist durch das Anschlagelement 58 auf der wendelförmigen Grundbahn 57 definiert, das dem Kolbenstangenanschlag 65 gegenüberliegt.

Damit kann in einfacher Art und Weise das maximale Ausspritzvolumen festgelegt werden, so dass damit sichergestellt ist, dass ein Benutzer stets das festgelegte Volumen des im Spritzenzylinder befindlichen Fluids über das vordere Abgabeende 4 abgeben kann.

Bei der hier beschriebenen Ausführungsform können beispielsweise die Anschlagflächen 59 von unmittelbar benachbarten Anschlagelementen 1,3 mm in Verschieberichtung 10 voneinander beabstandet sein, wodurch das Ausspritzvolumen in 0,5 ml-Schritten von 0,5 ml bis 10 ml einstellbar ist.

## Patentansprüche

1. Veterinärmedizinische Spritze mit
einem Grundkörper (2), der einen ein vorderes Abgabeende (4) und ein hinteres Ende (11) aufweisenden Spritzenzylinder (3) zur Aufnahme von abzugebendem Fluid umfasst, einer Kolbenstange (8), die einen vorderen Endabschnitt (9) mit einem Kolben (K), der im Spritzenzylinder (3) entlang einer Verschieberichtung (10) verschiebbar angeordnet ist, und einen hinteren Endabschnitt (12), der außerhalb des Spritzenzylinders (3) positioniert ist, aufweist,
einem Handgriff (13), der einen vorderen Griffteil (14) am Grundkörper (2) und einen hinteren Griffteil (15), der schwenkbar am vorderen Griffteil (14) befestigt ist, aufweist, und einer Dosiereinrichtung (50) zur Einstellung des abzugebenden Fluidvolumens, wobei der hintere Endabschnitt (12) der Kolbenstange (8) schwenkbar am hinteren Griffteil (15) befestigt ist, so dass durch Schwenken des hinteren Griffteils (15) in Richtung zum Grundkörper (2) hin die Kolbenstange (8) und somit der Kolben (K) im Spritzenzylinder (3) zum vorderen Abgabeende (4) bewegt wird, um im Spritzenzylinder (3) vorhandenes Fluid abzugeben,
wobei die Dosiereinrichtung (50) am Grundkörper (2) angeordnet ist und einen verstellbaren vorderen Anschlag (58) für die Kolbenstange (8) bereitstellt, der einen minimalen Abstand zwischen dem Kolben (K) und dem vorderen Abgabeende (4) festlegt, der beim Bewegen des Kolbens (K) im Spritzenzylinder (3) zum vorderen Abgabeende (4) erreicht werden kann,
wobei der verstellbare vordere Anschlag der Dosiereinrichtung (50) in mindestens zwei vorgegebene Einstellpositionen, in denen die Position des vorderen Anschlags (58) in Verschieberichtung (10) verschieden ist, bringbar ist, so dass durch das Einstellen einer der Einstellpositionen der maximale Hub der Kolbenstange (8) und damit das maximale abgebbare Fluidvolumen einstellbar ist,
**dadurch gekennzeichnet, dass**
der hintere Endabschnitt (12) der Kolbenstange (8) so schwenkbar am hinteren Griffteil (15) befestigt ist, dass eine relative Schwenkbewegung zwischen der Kolbenstange (8) und dem hinteren Griffteil (15) die einzig mögliche Relativbewegung zwischen der Kolbenstange (8) und dem hinteren Griffteil (15) ist,
wobei die Kolbenstange (8) so geführt ist, dass der Kolben (K) im Spritzenzylinder (3) nur entlang der Verschieberichtung (10) verschiebbar ist,
und wobei die Kolbenstange (8) mit einer solchen Elastizität bereitgestellt ist, dass eine bei einem Verschwenken des hinteren Griffteils (15) hin zum vorderen Griffteil (14) am hinteren Endabschnitt (12) der Kolbenstange (8) auftretende Bewegungskomponente quer zur Verschieberichtung (10) durch ein Verbiegen der Kolbenstange (8) kompensiert wird.

2. Veterinärmedizinische Spritze nach Anspruch 1,
wobei der hintere Endabschnitt (12) der Kolbenstange (8) schwenkbar an einem zum vorderen Griffteil (14) weisenden Abschnitt (42) des hinteren Griffteils (15) befestigt ist.

3. Veterinärmedizinische Spritze nach Anspruch 1 oder 2,
wobei die vom Grundkörper (2) weg weisende Seite (41) des hinteren Griffteils (15) als durchgehende Fläche ausgebildet ist.

4. Veterinärmedizinische Spritze nach einem der obigen Ansprüche,
wobei die vom Grundkörper (2) weg weisende Seite (41) des hinteren Griffteils (15) in einem Abschnitt, der dem hinteren Endabschnitt (12) der Kolbenstange (8) gegenüberliegt, als durchgehende Fläche ausgebildet ist.

5. Veterinärmedizinische Spritze nach einem der obigen Ansprüche,
wobei die Dosiereinrichtung (50) ein am Grundkörper drehbar gelagertes Drehelement (51) aufweist, das für jede der vorgegebenen Einstellpositionen ein Anschlagelement (58) umfasst,
wobei die Anschlagelemente (58) in Drehrichtung des Drehelementes (51) voneinander beanstandet angeordnet sind, und
wobei durch Drehen des Drehelements (51) das gewünschte Anschlagelement (58) als vorderer Anschlag positionierbar ist.

6. Veterinärmedizinische Spritze nach Anspruch 5,
wobei die Anschlagelemente (58) auf einer wendelförmigen Grundbahn (57) angeordnet sind, die einen Kanal (56) umgibt, durch den sich die Kolbenstange (8) erstreckt.

7. Veterinärmedizinische Spritze nach Anspruch 5 oder 6,
wobei das Drehelement (51) einen zylinderförmigen Rasterabschnitt (53) mit Nuten (60) aufweist,
wobei jede Nut (60) einem der Anschlagelemente (58) zugeordnet ist und der Grundkörper (2) ein Eingriffselement (62) aufweist, das bei den vorgegebenen Einstellpositionen in Eingriff mit einer der Nuten (60) steht.

8. Veterinärmedizinische Spritze nach einem der obigen Ansprüche,
wobei die Kolbenstange (8) einen Kolbenstangenanschlag (65) aufweist, der beim Erreichen des minimalen Abstands in Kontakt mit dem vorderen Anschlag (58) steht.

9. Veterinärmedizinische Spritze nach einem der obigen Ansprüche,
wobei am hinteren Griffteil (15) ein Anschluss (26) zum Verbinden eines Fluidreservoirs mit dem hinteren Griffteil (15) vorgesehen ist, wobei von dem Anschluss (26) durch einen Zuführkanal (20) in der Kolbenstange (8) eine erste Fluidverbindung bis zum Spritzenzylinder (3) vorliegt.

10. Veterinärmedizinische Spritze nach Anspruch 9,
wobei die Kolbenstange (8) in ihrem vorderen Endabschnitt (9) ein Rückschlagventil (22) aufweist, das die erste Fluidverbindung sperrt und nur öffnet, wenn der Druck im Zuführkanal (20) um einen vorbestimmten Wert über dem Druck im Spritzenzylinder (3) liegt.

11. Veterinärmedizinische Spritze nach einem der vorherigen Ansprüche,
wobei das vordere Abgabeende (4) über einen Abgabekanal (5) mit einem vorderen Ende (7) der Spritze (1) in einer zweiten Fluidverbindung steht und
im Abgabekanal (5) ein Rückschlagventil (6) angeordnet ist, das die zweite Fluidverbindung sperrt und nur öffnet, wenn der Druck im Spritzenzylinder (3) einen vorbestimmten Wert übersteigt.

12. Veterinärmedizinische Spritze nach einem der obigen Ansprüche,
wobei die Spritze als Selbstfüllerspritze ausgebildet ist.

## Claims

1. Veterinary syringe, having
a main body (2), which comprises a syringe cylinder (3), having a front dispensing end (4) and a rear end (11), for receiving fluid to be dispensed,
a plunger rod (8), which has a front end portion (9) with a plunger (K), which is arranged displaceably along a displacement direction (10) in the syringe cylinder (3), and a rear end portion (12), which is positioned outside the syringe cylinder (3),
a handle (13), which has a front gripping part (14) on the main body (2) and a rear gripping part (15), which is fastened pivotably to the front gripping part (14),
and a metering device (50) for setting the fluid volume to be dispensed,
wherein the rear end portion (12) of the plunger rod (8) is fastened pivotably to the rear gripping part (15) such that, by pivoting of the rear gripping part (15) in the direction of the main body (2), the plunger rod (8), and therefore the plunger (K), is moved in the syringe cylinder (3) to the front dispensing end (4) in order to dispense fluid present in the syringe cylinder (3),
wherein the metering device (50) is arranged on the main body (2) and provides an adjustable front stop (58) for the plunger rod (8), said stop defining a minimum distance between the plunger (K) and the front dispensing end (4), that can be reached when the plunger (K) is moved in the syringe cylinder (3) to the front dispensing end (4),
wherein the adjustable front stop of the metering device (50) can be brought into at least two predetermined setting positions, in which the position of the front stop (58) in the displacement direction (10) differs, and therefore, by means of the setting of one of the setting positions, the maximum stroke of the plunger rod (8), and therefore the maximum fluid volume which can be dispensed, can be set,
**characterized in that**
the rear end portion (12) of the plunger rod (8) is fastened pivotably to the rear gripping part (15) such that a relative pivoting movement between the plunger rod (8) and the rear gripping part (15) is the only possible relative movement between the plunger rod (8) and the rear gripping part (15),
wherein the plunger rod (8) is guided in such a manner that the plunger (K) is displaceable in the syringe cylinder (3) only along the displacement direction (10), and wherein the plunger rod (8) is provided with such elasticity that a movement component transversely with respect to the displacement direction (10) occurring at the rear end portion (12) of the plunger rod (8) when the rear gripping part (15) is pivoted towards the front gripping part (14) is compensated for by bending of the plunger rod (8).

2. Veterinary syringe according to Claim 1,
wherein the rear end portion (12) of the plunger rod (8) is fastened pivotably to a portion (42) of the rear gripping part (15) that faces the front gripping part (14).

3. Veterinary syringe according to Claim 1 or 2,
wherein the side (41) of the rear gripping part (15) that faces away from the main body (2) is configured as a continuous surface.

4. Veterinary syringe according to one of the above claims,
wherein the side (41) of the rear gripping part (15) that faces away from the main body (2) is configured as a continuous surface in a portion which lies opposite the rear end portion (12) of the plunger rod (8).

5. Veterinary syringe according to one of the above claims,
wherein the metering device (50) has a rotary element (51) which is mounted rotatably on the main body and comprises a stop element (58) for each of the predetermined setting positions,
wherein the stop elements (58) are arranged spaced apart from one another in the direction of rotation of the rotary element (51), and
wherein, by rotation of the rotary element (51), the desired stop element (58) can be positioned as the front stop.

6. Veterinary syringe according to Claim 5,
wherein the stop elements (58) are arranged on a helical main path (57) which surrounds a channel (56) through which the plunger rod (8) extends.

7. Veterinary syringe according to Claim 5 or 6,
wherein the rotary element (51) has a cylindrical latching portion (53) with grooves (60), wherein each groove (60) is assigned to one of the stop elements (58), and the main body (2) has an engagement element (62) which, in the predetermined setting positions, is in engagement with one of the grooves (60).

8. Veterinary syringe according to one of the above claims,
wherein the plunger rod (8) has a plunger rod stop (65) which, when the minimum distance is reached, is in contact with the front stop (58).

9. Veterinary syringe according to one of the above claims,
wherein a connection (26) for connecting a fluid reservoir to the rear gripping part (15) is provided on the rear gripping part (15), wherein there is a first fluid connection from the connection (26) through a feed channel (20) in the plunge rod (8) as far as the syringe cylinder (3).

10. Veterinary syringe according to Claim 9,
wherein the front end portion (9) of the plunger rod (8) has a nonreturn valve (22) which blocks the first fluid connection and opens only when the pressure in the feed channel (20) lies above the pressure in the syringe cylinder (3) by a predetermined value.

11. Veterinary syringe according to one of the above claims,
wherein the front dispensing end (4) is in a second fluid connection with a front end (7) of the syringe (1) via a dispensing channel (5) and a nonreturn valve (6) is arranged in the dispensing channel (5), said nonreturn valve blocking the second fluid connection and opening only when the pressure in the syringe cylinder (3) exceeds a predetermined value.

12. Veterinary syringe according to one of the above claims,
wherein the syringe is configured as a self-filling syringe.

## Revendications

1. Seringue vétérinaire comprenant
un corps de base (2) qui comprend un cylindre de seringue (3) possédant une extrémité avant de distribution (4) et une extrémité arrière (11) pour recevoir le fluide à distribuer,
une tige de piston (8) qui possède une partie d'extrémité avant (9) avec un piston (K) qui est disposé de manière coulissante dans le cylindre de seringue (3) le long d'une direction de coulissement (10), et une partie d'extrémité arrière (12) qui est positionnée à l'extérieur du cylindre de seringue (3),
une poignée (13) qui possède une partie de préhension avant (14) sur le corps de base (2) et une partie de préhension arrière (15) fixée de manière pivotante sur la partie de préhension avant (14),
et un dispositif de dosage (50) pour régler le volume de fluide à distribuer,
la partie d'extrémité arrière (12) de la tige de piston (8) étant fixée de manière pivotante à la partie de poignée arrière (15) de telle sorte que, par pivotement de la partie de poignée arrière (15) en direction du corps de base (2), la tige de piston (8) et donc le piston (K)
dans le cylindre de seringue (3) sont déplacés vers l'extrémité de distribution avant (4) afin de distribuer le fluide présent dans le cylindre d'injection (3),
le dispositif de dosage (50) étant disposé sur le corps de base (2) et prévoyant une butée avant réglable (58) pour la tige de piston (8), qui définit une distance minimale entre le piston (K) et l'extrémité de distribution avant (4), qui peut être atteinte lorsque le piston (K) se déplace dans le cylindre d'injection (3) vers l'extrémité de distribution avant (4),
la butée avant réglable du dispositif de dosage (50) pouvant être amenée dans au moins deux positions de réglage prédéfinies dans lesquelles la position de la butée avant (58) est différente dans le sens de déplacement (10), de sorte que le réglage de l'une des positions de réglage permet de régler la course maximale de la tige de piston (8) et donc le volume maximal de fluide pouvant être délivré,
**caractérisé en ce que**
la partie d'extrémité arrière (12) de la tige de piston (8) est fixée de manière pivotante à la partie de poignée arrière (15) de telle sorte qu'un mouvement de pivotement relatif entre la tige de piston (8) et la partie de poignée arrière (15) est le seul mouvement relatif possible entre la tige de piston (8) et la partie de poignée arrière (15),
la tige de piston (8) étant guidée de telle sorte que le piston (K) ne peut se déplacer dans le cylindre de pulvérisation (3) que dans le sens du déplacement (10),
et la tige de piston (8) étant pourvue d'une élasticité telle qu'une composante de mouvement apparaissant au niveau de la partie d'extrémité arrière (12) de la tige de piston (8) lors d'un pivotement de la partie de poignée arrière (15) vers la partie de poignée avant (14) est compensée par une flexion de la tige de piston (8) transversalement à la direction de déplacement (10).

2. Seringue vétérinaire selon la revendication 1 ,
la partie d'extrémité arrière (12) de la tige de piston (8) étant fixée de manière pivotante à une partie (42) de la partie de poignée arrière (15) tournée vers la partie de poignée avant (14).

3. Seringue vétérinaire selon la revendication 1 ou 2,
dans laquelle le côté (41) de la partie de poignée arrière (15) qui est opposé au corps de base (2) est réalisé sous forme de surface continue.

4. Seringue vétérinaire selon l'une des revendications précédentes,
dans laquelle le côté (41) de la partie de préhension arrière (15) qui est opposé au corps de base (2) est réalisé sous forme de surface continue dans une partie qui est opposée à la partie d'extrémité arrière (12) de la tige de piston (8).

5. Seringue vétérinaire selon l'une des revendications précédentes,
dans laquelle le dispositif de dosage (50) possède un élément rotatif (51) monté de manière rotative sur le corps de base, qui comprend pour chacune des positions de réglage prédéfinies un élément de butée (58),
les éléments de butée (58) étant disposés à distance les uns des autres dans le sens de rotation de l'élément rotatif (51), et
la rotation de l'élément rotatif (51) permettant de positionner l'élément de butée souhaité (58) comme butée avant.

6. Seringue vétérinaire selon la revendication 5,
les éléments de butée (58) étant disposés sur une trajectoire hélicoïdale (57) qui entoure un canal (56) à travers lequel s'étend la tige de piston (8).

7. Seringue vétérinaire selon la revendication 5 ou 6,
dans laquelle l'élément rotatif (51) possède une partie de trame cylindrique (53) avec des rainures (60),
chaque rainure (60) étant associée à l'un des éléments de butée (58) et le corps de base (2) possédant un élément d'engagement (62) qui, dans les positions de réglage prédéfinies, est en prise avec l'une des rainures (60).

8. Seringue vétérinaire selon l'une des revendications précédentes,
la tige de piston (8) possédant une butée de tige de piston (65) qui, lorsque la distance minimale est atteinte, est en contact avec la butée avant (58).

9. Seringue vétérinaire selon l'une des revendications précédentes,
dans laquelle un raccord (26) est prévu sur la partie arrière de la poignée (15) pour relier un réservoir de fluide à la partie arrière de la poignée (15), une première communication de fluide étant établie entre le raccord (26) et le cylindre de seringue (3) par un canal d'alimentation (20) dans la tige de piston (8).

10. Seringue vétérinaire selon la revendication 9,
dans laquelle la tige de piston (8) possède dans sa partie d'extrémité avant (9) un clapet anti-retour (22) qui bloque la première communication de fluide et ne s'ouvre que lorsque la pression dans le canal d'alimentation (20) est supérieure d'une valeur prédéterminée à la pression dans le cylindre de seringue (3).

11. Seringue vétérinaire selon l'une des revendications précédentes,
dans laquelle l'extrémité de distribution avant (4) est en communication fluidique avec une extrémité avant (7) de la seringue (1) par l'intermédiaire d'un canal de distribution (5) et un clapet anti-retour (6) est disposé dans le canal de distribution (5), qui bloque la deuxième connexion fluidique et ne s'ouvre que lorsque la pression dans le cylindre de seringue (3) dépasse une valeur prédéterminée.

12. Seringue vétérinaire selon l'une des revendications précédentes,
la seringue étant conçue comme une seringue à remplissage automatique.
